# EUROPEAN PATENT APPLICATION

(11) **EP 0 785 282 A2**
(43) Date of publication of application: **23.07.1997**
(21) Application number: 97300323.9
(22) Date of filing: 17.01.1997
(51) Int. Cl.: C12Q 1/68

(54) **Diagnosing and prognosing disease using sequences of the presenilin-1 gene**

(30) Priority: 19.01.1996 US 10241
(71) Applicant: WASHINGTON UNIVERSITY SCHOOL OF MEDICINE, St. Louis, Missouri 63110-1093 (US); UNIVERSITY OF SOUTH FLORIDA, Tampa, FL 33620-6250 (US); SMITHKLINE BEECHAM PLC, Brentford, Middlesex TW8 9EP (GB)
(72) Inventor: Goate, Alison M., Washington Univ. School of Med., St Louis, Missouri 63110 (US); Hardy, John A., Jacksonville, Florida 32224 (US); Roberts, Gareth W., SmithKline Beecham Pharma., Harlow, Essex CM19 5AW (GB)
(74) Representative: Giddings, Peter John, Dr.

(57) **Abstract**

A method of prognosing in a subject for the course and nature of disease progression and the ultimate degree of dementia which a subject would suffer during a b amyloid-related disease, or chronic neurodegenerative pathology due to brain pathology (e.g., hemorrhage) and head injury, wherein the presence of a PS-1 isoform or DNA in a subject at risk of such disease or suspected of having such a disease or in the early stages of such a disease indicates that the subject is likely to have a milder, more benign course of disease with the extent of the eventual cognitive impairment or dementia being of mild or moderate degree, the method comprising detecting the presence or absence of PS-1 polymorphisms or of DNA encoding PS-1 polymorphisms in the subject, particularly Inronic polymorphisms.

SEQUENCE LISTING

(1) GENERAL INFORMATION

(i) APPLICANT
(A) NAME: Washington University School of Medicine
(B) STREET: 660 South Euclid Avenue
(C) CITY: St. Louis
(D) STATE OR PROVINCE: Missouri
(E) COUNTRY: USA
(F) POSTAL CODE: 63110

(ii) TITLE OF THE INVENTION: Diagnosing and Prognosing
Disease Using Sequences of the
Presenilin-1 Gene

(iii) NUMBER OF SEQUENCES: 22

(iv) COMPUTER-READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(v) CURRENT APPLICATION DATA:
(A) APPLICATION NUMBER:

(2) INFORMATION FOR SEQ ID NO:1:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:1:

GTTTGTTTCT GCTTAATGTA 20

(2) INFORMATION FOR SEQ ID NO:2:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:2:

GTTTTTTCTT TCCCTTTTCA G 21

(2) INFORMATION FOR SEQ ID NO:3:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:3:

GTACAGTGT 9

(2) INFORMATION FOR SEQ ID NO:4:

(i) SEQUENCE CHARACTERISTICS:
(A) LENGTH: 20 base pairs
(B) TYPE: nucleic acid

(C) STRANDEDNESS: single
(D) TOPOLOGY: linear

(ii) MOLECULE TYPE: Genomic DNA

(xi) SEQUENCE DESCRIPTION: SEQ ID NO:4:

TGTTTTTCTT GTGCTTATAG 20

(2) INFORMATION FOR SEQ ID NO:5:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:5:

CGTATGAGAT TTGTTTT 17

(2) INFORMATION FOR SEQ ID NO:6:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:6:

TTGTGTTTGT TTTATTGTAG 20

(2) INFORMATION FOR SEQ ID NO:7:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:7:

GTGAGCATGA GACACAGATC 20

(2) INFORMATION FOR SEQ ID NO:8:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:8:

TGAAATGCTT TCTTTTCTAG 20

(2) INFORMATION FOR SEQ ID NO:9:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:9:

GTAAAACCCA AGACTGATAA 20

(2) INFORMATION FOR SEQ ID NO:10:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:10:

TTATGTTTTT CTTTTTCTAG 20

(2) INFORMATION FOR SEQ ID NO:11:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:11:

GTATGTGCAT TTCTCTATGT 20

(2) INFORMATION FOR SEQ ID NO:12:

(i) SEQUENCE CHARACTERISTICS:
(A) LENGTH: 20 base pairs

(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:12:

TTGTAACCTT TCCTTTTTAG 20

(2) INFORMATION FOR SEQ ID NO:13:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:13:

GTATGTGCAT TTCTCTATGT 20

(2) INFORMATION FOR SEQ ID NO:14:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:14:

TTGTAACCTT TCCTTTTTAG 20

(2) INFORMATION FOR SEQ ID NO:15:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:15:

GTAAGTATAC ACTAATAAGA 20

(2) INFORMATION FOR SEQ ID NO:16:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:16:

CTTTCCCATC TTCTCCACAG 20

(2) INFORMATION FOR SEQ ID NO:17:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:17:

CACCCATTTA CAAGTTTAGC 20

(2) INFORMATION FOR SEQ ID NO:18:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:18:

CACTGATTAC TAATTCAGGA TC 22

(2) INFORMATION FOR SEQ ID NO:19:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:19:

CCCAACCATA AGAAGAACAG 20

(2) INFORMATION FOR SEQ ID NO:20:

(i) SEQUENCE CHARACTERISTICS:
(A) LENGTH: 20 base pairs

(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:20:

GTGGTAATGT GGTTGGTGAT 20

(2) INFORMATION FOR SEQ ID NO:21:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:21:

AACAATGGTG TGGTTGGTGA 20

(2) INFORMATION FOR SEQ ID NO:22:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:22:

AAGTTTTGAC ATTAAGAGCT 20

## Description

### BACKGROUND OF THE INVENTION

### I.

Alzheimer's disease (AD) is a progressive degenerative disease of the central nervous system characterized clinically by dementia and neuropathologically by the presence of numerous senile plaques and neurofibrillary tangles. AD is typically a late onset disease of the elderly. However, a small number of pedigrees have been described wherein an early onset form of the disease is inherited as an autosomal dominant with age dependent penetrance. Most commonly, the age of onset of the disease is below 60 years old. Genetic factors have been implicated in both early and late onset AD.

Mutations in at least four different genetic loci are associated with an inherited susceptibility to AD. The e4 allele of the apolipoprotein E (ApoE) gene on chromosome 19 is associated with late onset AD (Strittmatter et al. Proc. Natl. Acad. Sci. USA 1993, 90:1977-1981; Saunders et al. Neurology 1993, 43:1467-1472); Corder et al. Science 1993, 261:921-923). Mutations in the B-amyloid (beta) precursor protein (BAPP) gene on chromosome 21 have been found in a small number of families with early onset AD (Goate et al. Nature 1991, 349:704-706; Chartier-Harlin et al. Nature 1991, 353:844-846; Murrell et al. Science 1991, 254:97-99; Karlinsky et al. Neurology 1992, 42:1445-1453). Most recently, a novel AD locus in a gene referred to as PS-2 was identified on chromosome 1 (1q31-41) from genetic linkage analysis of a "Volga German" kindred (Ephrat Levy-Lahad et al. Science 1995, 269:970-973; ibid. 973-977). The PS-2 gene bears a similarity to the AD-associated gene, PS-1.

Another locus (AD3) has been mapped by genetic linkage studies to chromosome 14q24.3 and may account for up to 70% of early-onset autosomal dominant AD (Schellenberg et al. Science 1992, 258:668-670; George-Hyslop et al. Nature Genet. 1992, 2:330-334; Van Boreckhoven et al. Nature Genet. 1992, 2:335-339). The AD3 locus is associated with the most aggressive form of this disease (onset between 30 and 60 years of age) and it has been suggested that mutations at this locus put into effect a biologically fundamental process leading to AD.

A novel gene with five missense mutations in seven pedigrees segregating early-onset autosomal dominant AD at the AD3 locus, the presenilin (herein "PS-1") gene was described by Sherrington et al. Nature 1995, 375:754-760. Analysis of the nucleotide sequence of the PS-1 transcript revealed heterozygous nucleotide substitutions in the reverse transcriptase-polymerase chain reaction products from affected members of six large pedigrees. The putative open reading frame (ORF) of PS-1 encodes a protein predicted to be a membrane protein and the pedigree-associated nucleotide substitutions change the encoded amino acids in transmembrane (TM) helices II(L146M), VI(E246A), and VII(Y410C) and in loops between TMII-TMIII(R163H) and TMVI-TMVII(V286L).

Mutations in the (PS-1) gene on chromosome 14 have been shown to cause a significant proportion of early onset, autosomal dominant AD (Sherrington, et al. Nature 1995; 375: 754-760). A large number of mutations have been described which cause disease between the ages of about 30 and 50 years (Sherrington, et al. Nature 1995; 375: 754-760; Clark, et al. Nature Genetics 1995; 11: 219-222; Wasco, et al. Nature Medicine 1995; 1: 848). While conventionally, the PS-1 gene has been thought only to be involved in the rare, familial early onset form of the disease, evidence for allele sharing between affected family members with late onset disease has also been observed (Schellenberg, et al. Am. J. Hum. Genet. 1993; 53: 619-628). This allele sharing was not found when standard maximum likelihood methods were used, but statistically significant evidence for allele sharing was found when the affected pedigree member (APM) method of genetic analysis was used. This combination of results suggested that the chromosome 14 locus was not behaving as an autosomal dominant in the late onset form of the disease (Schellenberg, et al. Am. J. Hum. Genet. 1993; 53: 619-628).

Genetic analysis of late onset AD has so far implicated two loci in the aetiology of this form of the disease: the apolipoprotein E (ApoE) gene and the (al-antichymotrypsin (AACT) gene. As much as 40-50% of the risk for late onset AD is attributable to alleles at the ApoE locus (Corder, E. et al. Science 1993, 261: 921-923.) ApoE-e4 is thought to increase risk of AD in a dose dependent manner whilst the ApoE-e2 allele may play a protective role (Corder, E. et al. Science 1993, 261: 921-923; Corder, E. et al. Nature Genetics 1994; 7: 180-183; Talbot, C. et al. Lancet 1994; 343: 1432-1433.) More recently, it has been reported that homozygosity at a polymorphism within the AACT gene may modify the ApoE risk for AD (Kamboh, M. I. et al. Nature Genetics 1995; 10: 486-488.0.)

A number of other mutants of the PS-1 gene have now been identified. Genomic analysis of the PS-1 gene has defined the intron-exon boundaries of the primary transcription unit. This has led to the development of a method for identification of PS-I polymorphisms, particularly intronic polymorphisms which are predictive of disease as well as elucidation of several splicing variants and proximal coding mutations. These mutant PS-1 sequences, particularly intronic sequences are useful in the early detection of mutant forms of the PS-1 gene, a gene which is usually associated with aggressive early-onset AD but is also involved in the late onset of this disease as well. Methods for using the mutant forms of the PS-1 gene alone or in combination with ApoE genes for the diagnosis and prognosis of disease is also provided herein.

### II.

Accidental or non-accidental head injuries are common events. The precise number of patients suffering a head injury are difficult to calculate exactly since the methods of defining and counting cases varies from country to country. However the relevant figures for the UK serve as a useful guide to the extent of the problem. In the UK some 300 persons per 100,000 of the population are admitted to hospital each year as a result of head injury. Of these patients 9 per 100,000 will die as a direct result of the severity of their injuries. Outcome surveys in the USA indicate that for every 100 head injury survivors up to 5 remain in a coma, up to 15 are still severely disabled six months after injury, 20 have minor psychiatric or psychological problems and the remaining 60 will make a good recovery. These figures give rise to an estimated population of some 500, 000 persons in the USA who have a persisting handicap as a result of trauma related head injury. The social and economic cost of dealing with the after effects of such injuries is large (D. W. Anderson, et al. Jour of Neurosurg, 1980, 53: Suppl. S1-S43; W. F. Caveness. In: Thompson R . Green J R (ed). Advances in neurology, Vol. 22, Complications of nervous system trauma. Raven Press, New York, 1979, p1; Roberts G. W., et al. Neuropsychiatric Disorders. Gower Medical press. London 1993; J.H. Adams. In: JH Adams & LW Duchen (Eds.), Greenfield Neuropathology, 5th edition 1992. Edward Arnold, London, Melbourne, Auckland. p106-152.).

The cause of this problem is the brain damage that occurs in up to 100% of patients who are admitted to hospital with a head injury (J.H. Adams. In: JH Adams & LW Duchen (Eds.), Greenfield Neuropathology, 5th edition 1992. Edward Arnold, London, Melbourne, Auckland. p106-152.). The damage arises from the physical effects of the trauma (such as swelling, herniation, haemorrhage, global or focal damage or compromise of the vascular supply, contusion, cranial and peripheral nerve damage, axonal injury and embolism (Roberts G. W., et al. Neuropsychiatric Disorders. Gower Medical press. London 1993; J.H. Adams. In: JH Adams & LW Duchen (Eds.), Greenfield Neuropathology, 5th edition 1992. Edward Arnold, London, Melbourne, Auckland. p106-152; J.H. Adams, et al. Histopathology 1989, **15**: 49-59. Anon editorial. Head trauma victims in the UK: undeservedly underserved. Lancet 1990, **335** 886-887) and also from the neurochemical consequences of the ischaemia which invariably accompanies physical brain damage (Roberts G. W., et al. Neuropsychiatric Disorders. Gower Medical press. London 1993; J.H. Adams. In: JH Adams & LW Duchen (Eds.), Greenfield Neuropathology, 5th edition 1992. Edward Arnold, London, Melbourne, Auckland. p106-152; J.H. Adams, et al. Histopathology 1989, **15**: 49-59; Lancet 1990, **335** 886-887.) Such injuries and subsequent damage are often widespread and can involve regions of the spinal cord, cranial and peripheral nerves in addition to the brain (D. W. Anderson, et al. Jour of Neurosurg, 1980, 53: Suppl. S1-S43; Roberts G. W., et al. Neuropsychiatric Disorders. Gower Medical press. London 1993; J.H. Adams. In: JH Adams & LW Duchen (Eds.), Greenfield Neuropathology, 5th edition 1992. Edward Arnold, London, Melbourne, Auckland. p106-152.).

In addition the brain damage caused by head injury also produces the risk of subsequent psychiatric and neurologic complications including epilepsy and chronic neuro-degenerative states (eg dementia pugilistica or punch drunk syndrome) (Roberts G. W., et al. Neuropsychiatric Disorders. Gower Medical press. London 1993; J.H. Adams. In: JH Adams & LW Duchen (Eds.), Greenfield Neuropathology, 5th edition 1992. Edward Arnold, London, Melbourne, Auckland. p106-152; J.H. Adams, et al. Histopathology 1989, **15**: 49-59. Anon editorial. Head trauma victims in the UK: undeservedly underserved. Lancet 1990, **335**: 886-887; JAN Corsellis, et al. Psychol Med 1973, **3**: 270-273; W.A. Lishman. In: Organic Psychiatry, 2nd edition, Blackwell Scientific, London, Oxford 1987; J.A. Mortimer, et al. Int. J Epidemiol 1991, **20**: S28; A.J. Roberts. Pitman, London (1969); R. Rudelli, et al. Arch Neurol, 1982, **39:** 570-575.)

The extent of brain damage caused by a head injury can vary markedly from patient to patient. Thus, the likelihood and degree of sustaining pathological brain damage in the immediate aftermath of the trauma and the risk of subsequent chronic neurodegeneration leading to epilepsy or a dementing condition vary also.

The pathophysiology of head injury has been investigated in an effort to determine the molecular mechanisms which enable the acute triggering event of a head injury to be transformed into a chronic neurodegenerative pathological process (G.W. Roberts. Lancet, 1988, **2** (8626-8627): 1456-1458; G.W. Roberts, et al J Neurol Neurosurg Psychiatry, 1990a, **53:** 373-378; S.M. Gentleman, et al. Progress in Brain Research, 1993 **96:** 237-246; G.W. Roberts, et al. Journal of Neurology, Neurosurgery and Psychiatry 1994; **57**: 419-425.).

Head-injured patients show increased levels of β amyloid precursor protein immunoreactivity ( S.M. Gentleman, et al. Progress in Brain Research, 1993 **96:** 237-246) and some 30% of head injured patients have evidence of b amyloid protein deposition (G.W. Roberts, et al. Journal of Neurology, Neurosurgery and Psychiatry 1994; **57:** 419-425). This deposition can occur within days of a single head injury. The eventual consequence of substantial numbers of b amyloid deposits is the emergence of a clinical syndrome of cognitive decline and increasing dementia (Roberts G. W., et al. Neuropsychiatric Disorders. Gower Medical press. London 1993; W.A. Lishman. In: Organic Psychiatry, 2nd edition, Blackwell Scientific, London, Oxford 1987.) Such deposits have been shown to be present in a number of dementing syndromes and these include AD, cortical Lewy body disease, Parkinson's disease and the Alzheimer-type disease in patients with Down's syndrome (Roberts G. W., et al. Neuropsychiatric Disorders. Gower Medical press. London 1993; W.A. Lishman. In: Organic Psychiatry, 2nd edition, Blackwell Scientific, London, Oxford 1987.) In addition b amyloid deposits are present in the brains of patients with vascular and cerebrovascular disease and these latter conditions can predispose or contribute to the above diseases (Roberts G. W., et al. Neuropsychiatric Disorders. Gower Medical press. London 1993; W.A. Lishman. In: Organic Psychiatry, 2nd edition, Blackwell Scientific, London, Oxford 1987.)

As described above, the PS-1 gene genotype has been shown to be an important determinant in the etiology of AD and it is believed the presence and number of PS-1 alleles alone and in conjunction with ApoE alleles being associated with increased risk and earlier ages of onset of disease in both familial cases linked to chromosome 19 and sporadic cases. However, the exact role of PS-1 in the pathology of Alzheimer-type disease is uncertain.

The exact relationship of various environmental factors like head injury to subsequent degenerative conditions like AD is uncertain. Although epidemiological studies provide some evidence for a link (J.A. Mortimer, et al. Int. J Epidemiol 1991, **20**: S28) the reason for the susceptibility to a chronic degenerative condition following head injury in some patients (W.A. Lishman. In: Organic Psychiatry, 2nd edition, Blackwell Scientific, London, Oxford 1987; J.A. Mortimer, et al. Int. J Epidemiol 1991, **20**: S28; A.J. Roberts. Pitman, London (1969); R. Rudelli, et al. Arch Neurol, 1982, **39**: 570-575; G.W. Roberts, et al. Journal of Neurology, Neurosurgery and Psychiatry 1994; **57**: 419-425) is unknown at present. (See Nicoll et al, 1995)

Methods of diagnosing and prognosing AD have been described (WO 94/09155) based upon detecting (directly or indirectly) the presence or absence of certain protein isoforms in a subject. Certain PS-1 alleles are described in the literature as outlined above and can be detected as described in WO 94/09155 to prognose and diagnose early- and late-AD as provided by the present invention.

The present invention is directed to, among other things, diagnostic compositions and methods to detect PS-1 alleles and to measure the frequency of PS-1 alleles in those individuals with b amyloid deposition following head injury, since it is predicted to be of the same high order as that seen in AD, while in those head injured individuals without b amyloid deposition, the PS-1 allele frequency is believed to be similar to that in non-AD controls.

This invention provides further explanation and diagnostic tests and compounds for the susceptibility of some patients to a chronic neurodegenerative pathological process following the types of brain damage (e.g., axonal shearing, swelling, herniation, hemorrhage and ischaemia) caused by a head injury.

### III.

b amyloid protein-related disease is a heterogeneous class of disorders characterized by the deposition within the brain of insoluble deposits of the b amyloid protein (Roberts, G. W. et al. Neuropsychiatric Disorders. Gower Medical press. London 1993). The eventual consequence of substantial numbers of b amyloid deposits is the emergence of a clinical syndrome of cognitive decline and increasing dementia. Such deposits have been shown to be present in a number of dementing syndromes and these include AD, cortical Lewy body disease, Parkinson's disease and the Alzheimer-type disease in patients with ("DS"). In addition b amyloid deposits are present in the brains of patients with vascular and cerebrovascular disease these latter conditions can predispose or contribute to the above diseases (Roberts, G. W. et al. Neuropsychiatric Disorders. Gower Medical press. London 1993).

Neuropathological studies have shown that all individuals with Downs syndrome DS (trisomy 21) develop the pathological hallmarks of AD, i.e., b amyloid protein deposition, neurofibrillary tangle formation and neuronal cell loss (Adams, J. H. et al (eds). Greenfields Neuropathology 5th Ed. Edward Arnold, London 1992; DMA Mann, et al. Neuropath. App. Neurobiol. 1984, **10**: 185-207; MJ Ball et al. In Epstein CJ (ed). The neurobiology of Down's syndrome. New York Raven Press 1986, 45-58.). The extent of this pathology varies from patient to patient for reasons which are unknown and the extent of pathology in a given patient is unpredictable (C Oliver, et al. Down's syndrome and AD: A review Psychol. Med. 1986, **16**: 307-322; A. Johanson et al. Dementia 1991, **2**: 159-168; M B Schapiro et al. Nature of Mental Retardation and Dementia in Down Syndrome: Study With PET, CT, and Neuopsychology. Neurology of Aging. 1992; **13:** 723-734; H M Evenhuis. Arch Neurol. 1990, **47**: 263-267; MB Schapiro et al. Neurology 1989, **39**: 1349-1353).

Frequent clinical anecdotes have suggested that the occurrence of dementia in elderly cases of DS is not universal and that the degree of dementia can vary substantially (C Oliver, et al. Down's syndrome and AD: A review Psychol. Med. 1986, **16**: 307-322; A. Johanson et al. Dementia 1991, **2**: 159-168; M B Schapiro et al. Nature of Mental Retardation and Dementia in Down Syndrome: Study With PET, CT, and Neuopsychology. Neurology of Aging. 1992; **13**: 723-734). In this respect the variability seen in patients with Downs syndrome parallels the clinical heterogeneity of AD. However, recent studies have indicated that the development of such pathology and the associated dementia are significantly correlated to increasing age in the patient (M C Royston et al. Neurodegeneration 1994; **3:** 43-52).

The occurrence of AD in DS (trisomy 21) is usually ascribed to the effects of overproduction of b amyloid caused by a gene dosage effect on the amyloid precursor protein (APP) carried on chromosome 21. Mutations in this gene, some of which lead to overproduction of b amyloid from APP, are one cause of AD (Roberts G. W., et al. Neuropsychiatric Disorders. Gower Medical press. London 1993).

The present invention predicts that in patients with Downs syndrome increasing age will be linked to increasing degrees of AD-type pathology and these in turn would be associated with an increasing degree of dementia, and provides diagnostic methods and compounds for such AD-type pathology. Thus, given the association of PS-1 with late onset AD, and the association of late onset AD with longevity it would be predicted that Down's patient with one or more PS-1 alleles would accumulate more AD-type pathology and thus have a more profound degree of dementia.

### SUMMARY OF THE INVENTION

An object of the present invention is to provide the sequences of the intron-exon boundaries of the PS-1 gene.

Another object of the present invention is to provide a method for the elucidation, detection, and diagnosis of mutations in PS-1 sequences, including both intronic sequences associated with splice variation and in the open reading frames proximal to these intron-exon boundaries of the PS-1 gene through use of mutant PS-1 sequences, particularly intronic sequences.

Yet another object of the present invention is to provide the sequence of novel PS-1 mutations and to provide a method for their detection and diagnosis of AD, including early- and late- onset AD, and to assess the progression of dementia.

Yet another object of the invention is a method of prognosing the course and degree of dementia in patients with β amyloid-related disease, in particular Alzheimer-type disease in individuals with DS.

Still another object of the invention is a method of prognosing the likelihood of neurodegenerative pathology and dementia in head-injured patients and their likelihood of recovery from coma.

A further object of the invention is a method of prognosing the likelihood of recovery from intracerebral hemorrhage

A still further object of the invention is a method of prognosing the likelihood of non-response to compounds which block or alter synaptic transmission.

### BRIEF DESCRIPTION OF THE FIGURES

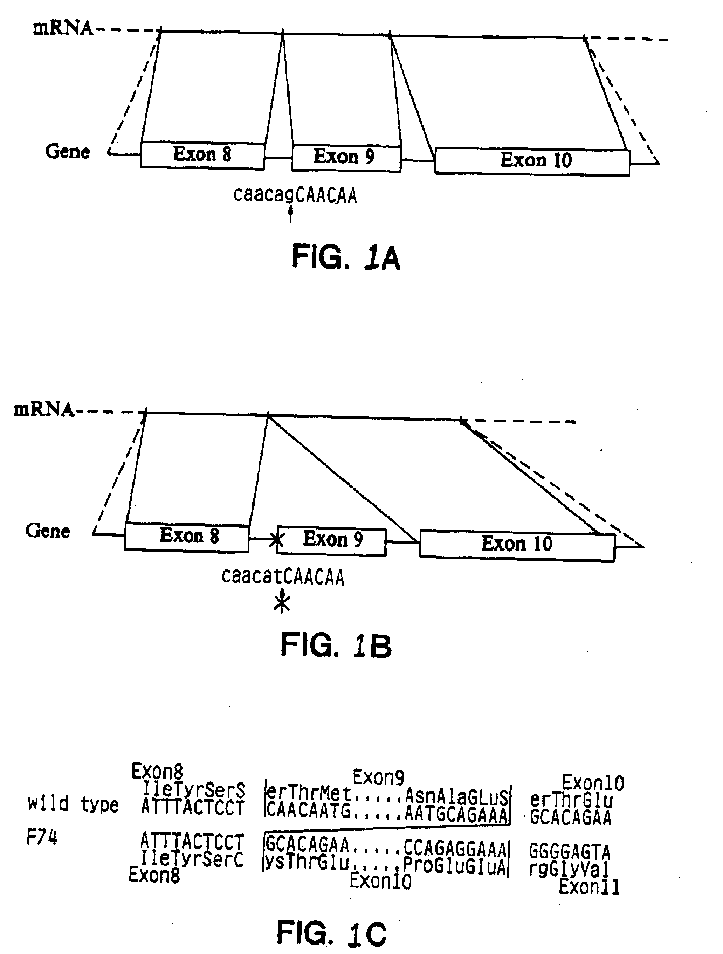

**Figure 1** provides diagrams of the location of the F74 (and possible F184) intronic mutations. Figure 1A shows the normal splicing of exon 9. Figure 1B shows the splicing of mutant PS-1 allele in F74 resulting in the loss of exon 9. Figure 1C provides a comparison of wild type and F74 mRNA and amino acid sequences.

### DETAILED DESCRIPTION OF THE INVENTION

Genetic linkage strategies placed a gene causing early onset familial AD (FAD) on the long arm of chromosome 14 between D14S289 and D14S61. Five mutations within the PS-1 gene, which map to this region were recently reported in several families multiply affected by early onset AD (between 30-50 years) (Sherrington et al. Nature 1995, 375:754-760). While conventionally this gene has been thought to only be involved in the rare, familial early onset form of the disease, some evidence for allele sharing between affected family members with late onset disease has also been observed (Schellenberg et al. Am. J. Hum. Genet. 1993, 53:619-628). This allele sharing between affected family members was not found when standard maximum likelihood methods were used, but was found when the affected pedigree member method of genetic analysis was used, suggesting that the locus was not behaving as an autosomal dominant in the late onset form of the disease.

During sequence analysis of early onset Alzheimer disease cases, a common polymorphism within the intron 3' to exon 9 of the PS-1 gene was identified (the term "intronic polymorphism(s)" as used herein refers to such polymorphism(s) within and proximal to the intron; certain of these polymorphisms affect splicing by, among other things, destroying splice donar, acceptor and/or branch sites, thereby altering the resulting protein, while other of these polymorphisms are silent, having no effect on the resulting protein). The most common allele has an A at nucleotide 16 (allele 1) in the intron while the variant allele has a C at this position (allele 2).

The present invention provides a method of detecting this and other polymorphisms utilizing mismatch PCR primers which introduce a BamHI site when the variant C is at nucleotide 16, but not when the A is present. This allows for the rapid analysis of a large number of samples using PCR followed by digestion with the restriction enzyme for BamHI and agarose gel electrophoresis.
Digestion refers to catalytic cleavage of a nucleic acid sequence with a restriction enzyme that acts only at certain sites in the sequence. Restriction enzymes such as that for BamHI are commercially available and their reaction conditions, cofactors and other requirements were used as would be known to the ordinarily skilled artisan. For analytical purposes, typically 1 mg of plasmid or DNA fragment is used with about 2 units of enzyme in about 20 ml of buffer solution. Appropriate buffers and substrate amounts for particular restriction enzymes are specified by the manufacturer. Incubation times of about 1 hour at 37° C are ordinarily used, but may vary in accordance with the supplier's instructions. After digestion the reaction is electrophoresed directly on a agarose gel to isolate the desired fragment.

Size separation of the cleaved fragments is performed using 8 percent polyacrylamide gel described by Goeddel, D. et al., Nucleic Acids Res. 1980, 8:4057.

The PS-1 polymorphisms of the invention, particularly the intronic polymorphisms will also be prognostic and diagnostic of early- and late-onset AD, dementia in patients with β amyloid-related disease, neurodegenerative pathology and dementia in head-injured patients, the likelihood of recovery from intracerebral hemorrhage, the likelihood of recovery from coma, and the likelihood of non-response to compounds which block or alter synaptic transmission.

The method of the present invention was used to screen for the presence of this polymorphism in a clinical series of AD cases and age matched controls. This polymorphism shows a strong association with the occurrence of typical late onset AD, with homozygosity for the more common of the two alleles causing an apparent doubling of the risk for this form of the disease (see Table 2 in Example 9c). Table 2 illustrates the association of the presence of an intronic polymorphism in homozygous form with the occurrence of typical late onset AD.

This polymorphism indicates that the PS-1 gene is involved in the pathogenesis of at least a proportion of late onset AD cases. Furthermore, the polymorphism may act by giving rise to alternative splicing in this area; however, whether it acts by this or another mechanism in no way limits the scope of the invention.

Intronic sequences in other areas of the PS-1 are believed to be important for similar reasons. A full-length clone of PS-1 was isolated from human cerebellar cDNA libraries. The entire intron-exon structure of this PS-1 gene was determined by comparison of cDNA and genomic DNAs (Figure 1). The PS-1 intronic sequences and their reverse complements near the intron-exon boundaries for the 12 exons were determined and are shown in Table 1.

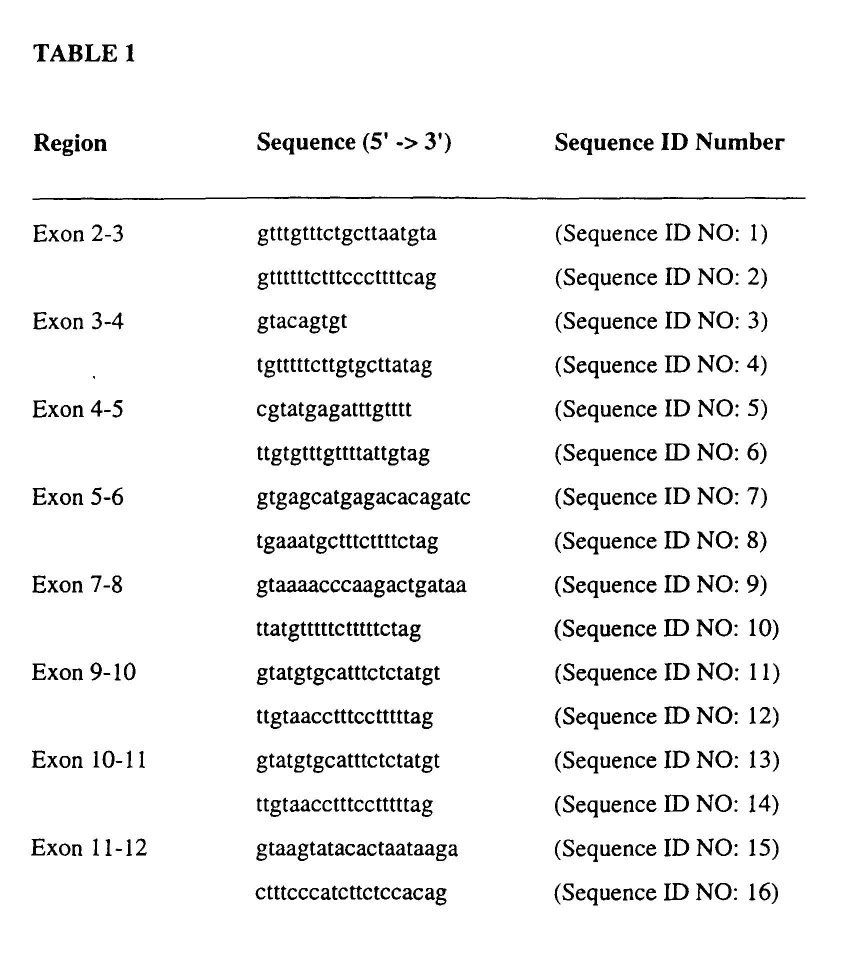

Analysis of the intron-exon boundaries of the novel PS-1 gene indicated that a novel alternative splicing variant of the PS- 1 gene had been expressed. Furthermore, using these primers, a mutation in the last nucleotide of the intron between exons 8 and 9 was found in at least one early onset AD family (F74) (Figure 1). A novel mutation was identified in exon 5 which would have been impossible to detect without intronic sequences. Identification and analysis of mutants or variants arising from mutations in splice donor or acceptor sites are enabled by knowledge of these intronic sequences. Furthermore, a complete analysis of the intron-exon boundaries makes possible sequencing primers that would allow accurate sequence determination of the first or last 10 to 20 nucleotides of coding exons especially near cDNA termini.

It has now been found that two of the allele intronic polymorphisms just 3' of exon 9 in the PS-1 gene show a robust association with the occurrence of typical late onset AD (AD), with homozygosity for the more common of the two alleles causing an apparent doubling of the risk for this form of the disorder (OR=1.97, CI=1.29-3.00).

The proportion of AD cases in the Caucasian population that can be attributed to homozygosity at this locus, as estimated by the attributable fraction, is 22%. The entire open reading frame of PS-1 in ten homozygous individuals has been sequenced and failed to detect any mutations in the coding sequence, indicating that PS-1 is important in certain cases of late onset AD in addition to its role in early onset, familial disease. Homozygosity for allele 1 of the PS-1 gene polymorphism is associated with increased risk for development of AD, particularly in, but not limited to, the Caucasian population.

The position of the polymorphism in the intron between exons 8 and 9 indicates that the polymorphism's mode of action may be through the modulation of alternate splicing, particularly of exons 8 and 9 of the PS-1 gene; however whether this or another model is the mechanism of such mode of action in no way limits the scope of the invention. Exon 8 is known to be alternately spliced in_some tissues (Rogaev, E.I., et al. Nature 1995, 376, 775-778) and a mutation in the acceptor site in the same intron leads to early onset disease in a British family through the loss of exon 9 (Perez-Tur, J., et al. NeuroReport 1995 (in press)). In addition, exon 8 is the site of the most prominent cluster of mutations leading to early onset disease (Van Broeckhoven, C.M. Nature Genetics 1995; 11; 230-232). No gross effects on the polymorphism on alternate splicing in control and AD brain tissue has been observed. Moreover, since the data herein demonstrates that the association does not exist in all populations another model is that the polymorphism is in linkage disequalibrium with an etiologically relevant variability elsewhere in the PS-1 gene; however, whether this or some other model is the mechanism for the association in no way limits the scope of the invention. Sequence analysis of the open reading frame of 10 cases with a 11 genotype has failed to reveal coding changes in these samples. Thus, if there is other aetiologically relevant variability within the PS- 1 gene, it must be in the promoter or other non-coding regions.

In view of the present invention it is believed that all cases of AD share common pathogenic mechanisms and that PS-1 is part of this biochemical pathway.

The present invention provides a method of prognosing in a head-injured subject or a subject who may be at risk of sustaining a head injury for the likelihood that a head injury might give rise to a chronic neurodegenerative pathology which could result in neuropsychological, psychiatric or neurological deficits, the method comprising detecting the presence or absence of PS-1 polymorphisms, particularly intronic polymorphisms, or of DNA encoding such polymorphisms in the subject. This detection may be performed alone or in combination with detection of ApoE polymorphisms.

The step of detecting the presence or absence of PS-1 protein isoforms, particularly intronic polymorphism protein isoforms or of DNA encoding such isoforms may be carried out either directly or indirectly by any suitable means, such as by techniques well known in the art, and is preferably carried out *ex vivo* (eg by means of the method described). All generally involve the step of collecting a sample of biological material containing either DNA or protein from the subject, and then detecting which isoforms the subject possesses from that sample. For example, the detecting step may be carried out by collecting a PS-1 sample from the subject (for example, from cerebrospinal fluid, or any other fluid or tissue containing or predicted to contain PS-1), and then determining the presence or absence of a PS-1 protein isoform, particularly an intronic polymorphism protein isoform in the PS-1 sample (e.g., by isoelectric focusing or immunoassay). In the alternative, the detecting step may be carried out by collecting a biological sample containing DNA from the subject, and then determining the presence or absence of DNA encoding a PS-1 protein isoform, particularly an intronic polymorphism protein isoform in the biological sample. Any biological sample which contains the DNA of that subject may be employed, including tissue samples and blood samples, with blood cells being a particularly convenient source. Determining the presence or absence of DNA encoding a PS-1 protein isoform, particularly an intronic polymorphism protein isoform may be carried out with an oligonucleotide probe labeled with a suitable detectable group, or by means of an amplification reaction such as a polymerase chain reaction or ligase chain reaction (the product of which amplification reaction may then be detected with a labeled oligonucleotide probe). Further, the detecting step may include the step of detecting whether the subject is heterozygous or homozygous for the gene encoding a PS-1 protein isoform, particularly an intronic polymorphism protein isoform. Numerous different oligonucleotide probe assay formats are known which may be employed to carry out the present invention. Suitable examples of techniques and strategies for detecting the PS-1 isoforms of the invention and encoding DNA are to be carried out as generally described in WO 94/09155.

It will be readily appreciated that the detecting steps may be carried out directly or indirectly. Using the above methods skilled artisans will readily be able to determine if an individual is homozygous or heterozygous for any given allele of PS-1, particularly intronic polymorphisms, alone or in combination with ApoE alleles.

The present invention has utility in enabling improvements in the clinical prognosis of patients who have suffered a degree of brain damage following a head injury.

In addition the invention has utility in allowing definition of the degree of risk in individuals who may be at risk of sustaining a head injury through social or professional activities (eg amateur and professional boxers, divers and other sportsmen such as rugby players, football players, mountain climbers, judo players etc) or through elective medical procedures known to be associated with increased risk of brain damage (eg cardiac bypass operations, carotid endardectomy, brain surgery etc).

The method of the invention may thus be used to determine the degree of risk in participating in sporting events or clinical procedures. Such prognostications will have considerable utility in the design, planning and implementation of clinical care for patients in the event of a head injury and in the appropriate therapeutic intervention or the degree of hospital/social intervention or support required by a patient deemed to be at greater risk of a neurodegenerative disorder or in the design and analysis of clinical trials to determine the efficacy of therapeutic agents in the treatment of the types of brain damage which occur following head injury.

The present invention also provides a method of prognosing in a subject for the course and nature of disease progression and the ultimate degree of dementia which a subject would suffer during a b amyloid-related disease, wherein the presence of an PS-1 isoform, particularly an intronic polymorphism isoform, or DNA encoding such isoforms in a subject at risk of such disease or suspected of having such a disease or in the early stages of such a disease indicates that the subject is likely to have a milder, more benign course of disease with the extent of the eventual cognitive impairment or dementia being of mild or moderate degree, the method comprising detecting the presence or absence of PS-1 protein isoforms, including intronic polymorphism protein isoforms, or of DNA encoding such proteins isoforms in the subject.

Suitable subjects include those which have not previously been diagnosed as afflicted with b amyloid-related disease and which have previously been determined to be at risk of developing b amyloid-related disease, in particular DS individuals.

The method of the invention is applicable to patients at risk of, suffering from or suspected of suffering from b amyloid-related diseases such as early- and late-onset AD, cortical Lewy body disease, Parkinson's disease and patients with vascular and cerebrovascular disease which predispose to the above diseases.

Such prognostications will have considerable utility in the design, planning and implementation of clinical care for such patients or any patient with PS-1 related neurodegenerative disease. The method of the invention may be used to determine the appropriate therapeutic intervention or the degree of hospital/social intervention or support required by a patient, by enabling prognostication of the rate of progression of dementia and the degree of dementia. Such prognostications will be of use in, for example, interpreting and deciding on the efficacy or likely outcome of clinical treatments for the above diseases; potential for response to different types of therapeutic intervention; selection of patients for clinical trials; analysis of data from clinical trials; forecasting and planning the requirements for facilities required to care for patients with such disease; and may be used to sub-divide and categorize such patients in order to maximize the use of such facilities and resources.

In a further aspect, the invention provides a method of treatment of β amyloid-related disease by the supplementation of PS-1 protein expression, for example by direct administration of the non-mutant PS-1 isoform or by gene therapy, or by the generation of an agonist which mimics the action of PS-1 on the PS-1 receptor(s). Direct administration may be by injection of the protein. Gene therapy may be by way of delivery of the PS-1 gene or by enhancement of the expression of the patient's own PS-1 gene. Such methods are generally known in the art.

The following examples are provided for illustrative purposes only and are not intended to limit the invention.

### EXAMPLES

### Example 1: Screening protocol for the T > G polymorphism 3' of exon 9

A mismatch primer was designed which contained two mismatched base pairs four and five base pairs away from the 3' end of the primer and five and six base pairs from the polymorphism. When incorporated in a PCR product, this primer produces a BamHI cut when G is present and no cut when T is present at the polymorphism site. This enables the two alleles to be distinguished by digestion of a PCR product with BamHI. Forward primer is 5' CACCCATTTACAAGTTTAGC 3' (SEQ ID NO: 17) and the reverse primer is 5' CACTGATTACTAATTCAGGATC 3' (SEQ ID NO: 18). This produces a PCR product of 200 bp which is cleaved by BamHI to produce fragments of 182 and 18 bp. DNA (50-100 ng) was used as a template in 25 ml reactions. The reaction mix consisted of the following concentrations: 0.2 mM dNTP's, 30 pM primer, 1X TNK50 buffer, 0.5 U Taq DNA polymerase. PCR was carried out under the following conditions: 94°C for 5 minutes; 94°C for 30 seconds; 45°C for 30 seconds; 72°C for 30 seconds; x 35 cycles; 72°C for 3 minutes. 5 U of BamHI enzyme was added to the PCR products and digestion carried out at 37°C for 3 hours. The digested product was run on a 3% agarose gel which is sufficient to separate the digested products so that 200 bp and 182 bp bands can be distinguished.

### Example 2: Elucidation of a novel splice variant

Expression of an additional form of the PS-1 protein containing an insertion of four amino acids at codons 26-27 (VRSQ) was found. The VRSQ insertion arises from alternative use of a 5' exon donor site in the exon 3/intron 3 (-52 to 75 nt) boundary. (See Figure 1 and Table 1). The ...CAG/gta... boundary of the final Gln codon of exon 3 of the VRSQ motif provides the ideal 5' exon AG donor site and GT intron consensus 5' boundary and use of this splice site results in the insertion of the 12-nts encoding the VRSQ motif. The upstream ...ACT/GTA... boundary of the Thr-Val codons provides the less preferred CT (AG preferred) 5' exonic boundary to the consensus GT 5' intronic boundary and splicing at this site would remove the VRSQ motif. Sherrington et al. Nature 1995, 375:754-760, reports the expression of only the VRSQ minus form. However, screening of fibroblasts with probes covering this region have shown that both splice variants are represented. Primers from the intronic region upstream (forward) and downstream exonic region of this alternate splicing sites allowed for their sequencing and elucidation.

### Example 3: Elucidation of novel splicing between exon 8 and 9

A mutation has been found in an early onset AD family designated F74 in the last nucleotide of the intron between exons 8 and 9, G → T. This mutation spoils the acceptor site in the middle of Serine 857 and is expected to alter the splicing of this region. This mutation does not change the cDNA sequence and could, therefore, only be identified by sequencing of primers derived from intronic sequences.

### Example 4: Identification of E120K mutation (G → A)

Additionally, a novel mutation was identified in exon 5 of PS-1 at codon 120 (lys → glu) arising from a G to A transition (GAA → AAA). This mutation is near the second putative transmembrane domain (TM2). This mutation is virtually impossible to detect without intronic sequence primers, as it is within 20 bp of the intron-exon boundary in genomic clones. E120K was found by PCR. A 100 µl reaction mix contained water, 10X buffer, 10 mM dNTP's, taq polymerase and 20 µM primer (5'-CCCAACCATAAGAAGAACAG-3' (SEQ ID NO: 19) and 5'-GTGGTAATGTGGTTGGTGAT-3' (SEQ ID NO: 20)). The PCR conditions were 94°C for 5 minutes, (94°C for 30 seconds, 50°C for 30 seconds, 72°C for 45 seconds) x 35, 72°C for 10 minutes. The PCR products were electrophoresed on a 3% agarose gel and visualized by ethidium bromide. The biotinylated primer allows single stranded DNA to be derived using streptavidin-coated magnetic beads and sequenced on an ALF sequencer (Pharmacia) using Autoread kits (Pharmacia).

### Example 5: Identification of L250S mutation (T → C)

Family 184 was also screened for mutations by PCR. A 100 µl reaction mix contained water, 10X buffer, 5 mM dNTP's, taq polymerase and 20 µM primer (5'-AACAATGGTGTGGTTGGTGA-3' (SEQ ID NO: 21) and 5'-AAGTTTTGACATTAAGAGCT-3' (SEQ ID NO: 22)). The PCR conditions were 94°C for 5 minutes, (94°C for 30 seconds, 50°C for 30 seconds, 72°C for 45 seconds) x 35, 72°C for 10 minutes. The PCR products were electrophoresed on a 3% agarose gel and visualized by ethidium bromide. The biotinylated primer allows single stranded DNA to be derived using streptavidin-coated magnetic beads and sequenced on an ALF sequencer (Pharmacia) using Autoread kits (Pharmacia). The base change T to C was found at codon 250 changing leucine to serine, by comparison with the normal PS-1 sequence.

### Example 6: PS-1 variants in head injury patients

Individuals surviving for less than two weeks following a severe head injury are selected from a database having data on such individuals, such as the Glasgow head injury database (J.H. Adams. In: JH Adams & LW Duchen (Eds.), Greenfield Neuropathology, 5th edition 1992. Edward Arnold, London, Melbourne, Auckland. p106-152.). Immunostaining and/or genotyping for mutant PS-1 nucleic acids are performed by standard methods well known to those skilled in the art using the PS-1 sequences provided herein as probes or primers.

Allelic prevalence of the mutant allele as compared to normal individuals can be assessed using standard statistical and comparative methods.

### Example 7 : PS-1 genotyping of elderly DS patients

Mutant PS-1 genotypes are examined in a series of clinically assessed elderly cases of DS. The case notes of each patient can be retrospectively assessed to rate the degree of dementia.

PS-1 genotyping was carried out using a modification of standard procedures (M B Schapiro et al. Nature of Mental Retardation and Dementia in Down Syndrome: Study With PET, CT, and Neuopsychology. Neurology of Aging. 1992; **13**: 723-734)

Allelic prevalence of the mutant allele as compared to normal individuals can be assessed using standard statistical and comparative methods.

A study of patients with DS can be made to statistically determine the effects of mutant PS-1 allelle status on the age at death and presence of dementia.

Retrospective clinical assessment of the cases are made by analysis of the clinical notes to determine the presence or absence of a dementia state in the patients with DS. The Clinical assessments are carried out blind to the genotyping results.

PS-1 genotyping was carried out using a modification of standard procedures (M B Schapiro et al. Nature of Mental Retardation and Dementia in Down Syndrome: Study With PET, CT, and Neuopsychology. Neurology of Aging. 1992; **13:** 723-734).

Comparison of the genotypes with the clinical features of the disease in those DS cases who lived to greater than 50 years can be performed to determine the overall allele frequencies

### Example 8: Statistical Analysis

Data is analyzed using standard statistical methods to determine whether possession of an PS-1 allele has a significant effect on age at death and presence and degree of dementia (for example, see S.M. Gentleman, et al. Progress in Brain Research, 1993 96: 237-246).

### Example 9: Screening protocol for polymorphism

During sequence analysis of early onset AD cases a common polymorphism within the intron 3' to exon 9 was identified. The most common allele has an A at nucleotide 16 (allele 1) in this intron whilst the variant allele has a C at this position (allele 2) (see reference 2). The polymorphism does not create or destroy any restriction enzyme sites. A mismatch primer was designed which contained two mismatched basepairs four and five base pairs from the 3' end of the primer and five and six base pairs from the polymorphism. When incorporated into a PCR product this primer produces a BamHI cut site when a C is present and no cut site when an A is present at the polymorphic site. This enables the two alleles to be distinguished by digestion of a PCR product with BamHI. Forward primer 951 5' CACCCATTTACAAGTTTAGC 3', Reversed (mismatched) primer 5' CACTGATTACTAATTCAGGATC 3'. Use of these primers gives a PCR product of 200bp which is cleaved by BamHI to produce fragments of 182 and 18bp. DNA 50-100ng was used as template in 25ml reactions. The reaction mix consisted of the following final concentrations:- 0.2mM dNTP's, 30pM each primer, 1x TNKSO buffer, 0.5U Taq DNA polymerase. PCR was carried out under the following conditions 94°C 5 mins (94°C 30sec, 45°C 30sec, 72°C 30sec) x35 cycles, 72°C 3mins. 5U of BamHI enzyme were added to the PCR and digestion carried out at 37°C for 3 hours. The digested product was run out on a 3% agarose gel which is sufficient to separate the digested product so that 200bp and 182bp bands can be distinguished. Multiple samples were rechecked by sequencing to ensure that allele scoring system was accurate.

### A. Subjects

Four independent samples were collected and analyzed. First, Caucasian case/control samples were recruited from the Greater St. Louis metropolitan and second, a similar, though smaller, sample was collected from the Tampa metropolitan area. Third, the African American case/control sample was recruited at St. Louis as above. Recruitment for these three sample series was not based upon a prior family history of AD; however, approximately 40-50% of participants have a first degree relative with a diagnosis of dementia of the Alzheimer type (DAT). Diagnostic criteria for DAT are equivalent to or more stringent than those for probable and possible AD by the National Institute of Neurological and Communicative Disorders (NINCDS/ADRDA) Workshop (McKhann, G. et al. Neurology 1984; 34: 939-944). Clinical diagnostic accuracy for histological AD in these subjects is 96% (Berg, L. et al. (eds). AD. New York: Raven Press, 1994; 9-21). Control subjects for each group met all exclusionary criteria for DAT but were not demented. Blood samples were collected from 208 Caucasian DAT patients (mean age 76.9 yrs, s.d.8.8 yrs, 58% female) and 185 age-matched controls (mean age 76.2 yrs , s.d. 11.0 yrs, 58% female). The mean age of onset of dementia in the DAT patients was 71.3 yrs, s.d. 8.9 yrs. Blood samples were collected from 29 African American DAT patients (mean age 77.2 yrs, s.d. 9.6 yrs, female) and 50 age-matched controls (mean age 72.0 yrs , s.d. 7.8 yrs, female). The mean age of onset of dementia in the DAT patients was 71.8 yrs, s.d. 5.2 yrs. The fourth sample was collected in the UK as part of a study on the genetics of AD and has been previously described (Houlden, H. et al. Neurodegen. 1993; 2: 283-286). The 32 unrelated AD patients are Caucasian but come from families multiply affected by late onset AD (mean age 81.1 yrs, s.d. 7.3 yrs, 78% female). The mean age of onset of dementia in this sample was 73.4 yrs, s.d. 6.6 yrs. The polymorphism within intron 9 was used to test independently for association between the PS-1 gene and late onset AD in all four clinical samples, but since the ethnicity and genotype results for the St. Louis and Tampa Caucasian series were similar, these sample groups were pooled to increase the power of the analysis.

### B. Statistical Methods

Genotypic and allelic distributions were analyzed using a Pearson chi square test of association and, for 2x2 contingency tables, by calculating odds ratios. The effect of genotype on age at onset was examined using multiple linear regression. Logistic regression analyses were performed to examine the effect of gender, ApoE-e4 genotype, PS-1 11 genotype and interactions between these variables on predicting affection status. Two tailed tests of significance were used throughout. All analyses were performed using SPSS version 6 for Windows. Attributable fraction (AF) was calculated from the formula: AF = f(OR-1)/[1+f(OR-1)] where f is the frequency of the risk factor in the population and OR is the odds ratio for developing AD in individuals with the risk factor compared with those without the risk factor.

### C. Results

As there were no significant differences in genotype or allele distributions for the PS-1 polymorphism between Caucasian control and patient samples from St. Louis and Tampa a primary analysis was carried out using pooled control and pooled patient samples from these datasets (see Tables 2 and 3). The genotypic distribution in the control sample was close to that expected under Hardy-Weinberg equilibrium (chi=0.23, 1df, p=0.635). There are, however, significant differences in genotype (p=0.007) and allele (p=0.006) distributions between controls and patients in the Caucasian sample. The effect is entirely due to an increase in the proportion of individuals who are homozygous for the absence of the artificial BamHI site (i.e., individuals who are homozygous for allele A of Figure 1) amongst AD cases.

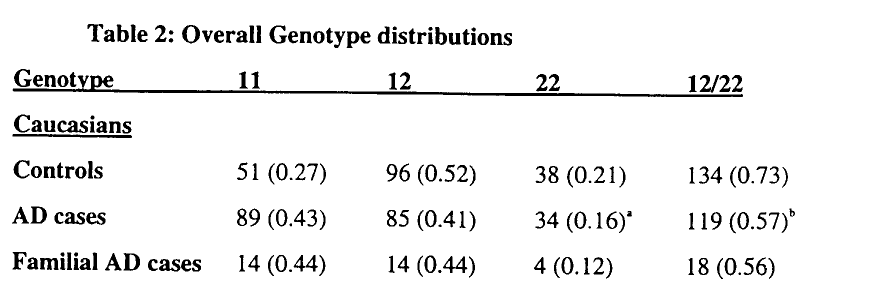

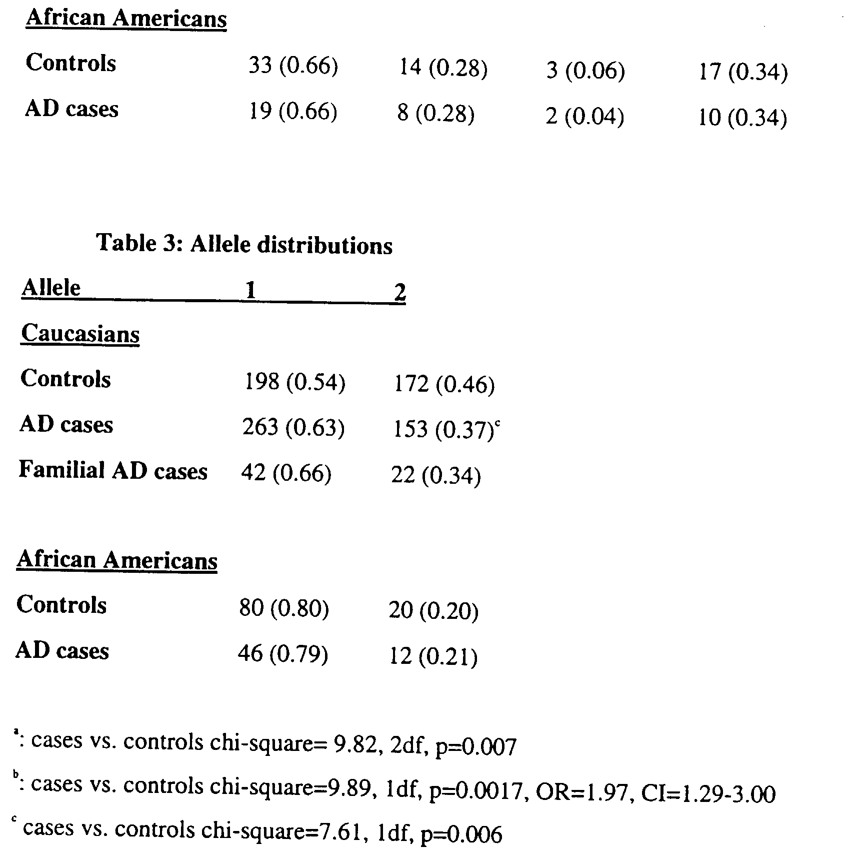

Using the 22 genotype as a reference, the 12 genotype is not associated with increased risk of AD (odds ratio, OR = 0.98, 95% confidence interval, CI = 0.57 - 1.68), whereas the 11 genotype is associated with an approximate doubling in risk (OR = 1.96, CI = 1.11 -3.48). In the analyses that follow, there are, therefore, combined the 12 and 22 genotypes into a single category to allow simple and more powerful comparisons between risk, as measured by the odds ratio, in carriers of the 11 genotype and those without this genotype (see Table 2).

Analysis of probands from the British late onset AD families gave essentially similar genotype frequencies to the American case series (Table 2) suggesting that the same effect might be operating in this sample, although the lack of an adequate control group for this sample precludes formal analysis of this sample set.

The ApoE genotypes of most of these samples have previously been determined and show the expected increase in ApoE-e4 allele frequency and decrease in age of onset of AD associated with ApoE-e4 dosage (Houlden, H. et al. Neurodegen. 1993; 2: 283-286). When the sample was split on the basis of PS-1 genotype, the PS-1 11 genotype was associated with a doubling of risk of AD in carriers of zero or one ApoE-e4 allele, but not in ApoE-e4 homozygotes (see Table 4). The small cell sizes for ApoE e4 homozygotes makes it difficult to interpret the significance of this observation. However, it is clear that for the common ApoE-e4 genotypes, there is no interaction between ApoE and PS-1 genotypes. There is no evidence of interaction between AACT and PS-1 in these data. However, in contrast to the report of Kamboh and colleagues (Talbot, C. et al. Lancet 1994; 343: 1432-1433) there is no obvious effect of AACT genotype on risk for AD in this dataset. (Talbot et al. Lancet 1994; 343: 1432-1433).

**Table 4**

| **ApoE-e4** | | **PS-1 genotype** | |
|---|---|---|---|
| **genotype** | | | |
| | | 11 | 12/22 |
| -/- | Control | 40 | 101 |
| | AD | 51 | 65^{a} |
| +/- | Control | 10 | 31 |
| | AD | 29 | 45^{b} |
| +/+ | Control | 1 | 1 |
| | AD | 7 | 7^{c} |
| ^{a}: Cases vs. controls: chi=6.77, 1df, p=0.009. OR = 1.98, CI = 1.18-3.33. ^{b}: Cases vs. controls: chi = 2.58, 1 df, p =0.108. OR = 2.00, CI = 0.85 - 4.00. ^{c}: Cases vs. controls: chi = 0.00, 1 df, p=1.00. OR = 1.00, CI = 0.05-19.4. | | | |

The data in Table 4 are derived from those illustrated in Table 2 except that ApoE genotype results were not available for 1 control and 4 AD cases which are thus omitted.

The association between the PS-1 11 genotype and AD is present and of similar magnitude for cases with an age of 65 years or less (OR = 1.84, CI = 0.97 - 3.50) and for cases with onset over the age of 65 years (OR = 2.10, CI = 1.32 - 3.33). Further, there was no evidence, using linear regression analyses (both including or excluding ApoE-e4 genotype as a variable), that PS-1 genotype predicts age of onset of dementia in this sample.

Logistic regression was used to build a model to describe the data (Hosmer DW, Lemeshow, S. "Applied logistic regression". 1989; New York: John Wiley & Sons). Consistent with the results presented above, ApoE-e4 genotype (p = 0.000003) and presence or absence of PS-1 11 genotype (p = 0.0027) were significant predictors of affection status. There was no significant interaction between PS-1 and PS-1 genotypes (p> 0.86). When logistic regression analyses were performed separately for males and females, the risk increasing effect of the PS-1 11 genotype was greater in females (OR = 2.24, CI = 1.21 - 4.15) than in males (OR = 1.71, CI = 0.86 - 3.14). However, this difference is not statistically significant, as demonstrated by the absence of significant interaction between PS-1 genotype and gender (p > 0.58) in the regression model on the total sample.

A measure of the overall contribution of a risk factor to disease in a population is provided by the attributable fraction, AF, which refers to the excess fraction of disease in a population that would not have occurred if the risk factor had been absent (Khoury MJ et al. "Fundamentals of genetic epidemiology". 1993; New York: Oxford University Press). For the PS-1 11 genotype in the sample, AF = 0.22. This compares with an AF of 0.35 for possession of a single copy of ApoE-e4 and 0.15 for two copies of ApoE-e4. Thus, in this sample PS-1 accounts for about half as much of the morbidity as does ApoE-e4.

Analysis of the small sample of African American cases and controls revealed that the frequency of this polymorphism was very different in the African American population as compared to the Caucasian sample but that the frequencies were similar between AD cases and controls (Tables 2 and 3).

## Claims

1. A method of prognosing in a head-injured subject or a subject who may be at risk of sustaining a head injury for the likelihood that a head injury might give rise to a chronic neurodegenerative pathology which could result in neuropsychological, psychiatric or neurological deficits, the method comprising detecting the presence or absence of PS-1 isoforms or of DNA encoding PS-1 isoforms in the subject.

2. A method according to claim 1 wherein the step of detecting the presence or absence of intronic polymorphisms or of DNA encoding such isoforms is carried out *ex vivo.*

3. A method according to claim 2 wherein said detection step involves collecting a sample of biological material containing DNA from the subject.

4. A method according to claim 3, wherein the biological sample is blood.

5. A method according to claim 2 wherein said detection step involves collecting a sample of biological material containing PS-1 from the subject.

6. A method according to claim 5, wherein the biological sample is cerebrospinal fluid.

7. A method of prognosing in a subject for the course and nature of disease progression and the ultimate degree of dementia which a subject would suffer during a b amyloid-related disease, wherein the presence of an PS- 1 isoform or DNA in a subject at risk of such disease or suspected of having such a disease or in the early stages of such a disease indicates that the subject is likely to have a milder, more benign course of disease with the extent of the eventual cognitive impairment or dementia being of mild or moderate degree, the method comprising detecting the presence or absence of intronic polymorphisms or of DNA encoding intronic polymorphisms in the subject.

8. A method according to claim 7 wherein the subject has not previously been diagnosed as afflicted with b amyloid-related disease.

9. A method according to claim 8 wherein the subject has previously been determined to be at risk of developing b amyloid-related disease.

10. A method according to any of claims 7 to 9 wherein the subject is at risk of, suffering from or suspected of suffering from AD.

11. A method according to any of claims 7 to 9 wherein the subject is at risk of, suffering from or suspected of suffering from cortical Lewy body disease.

12. A method according to any of claims 7 to 9 wherein the subject is at risk of, suffering from or suspected of suffering from Parkinson's disease.

13. A method according to any of claims 7 to 9 wherein the subject is at risk of, suffering from or suspected of suffering from vascular and cerebrovascular disease which predispose to b amyloid-related diseases.

14. A method according to claim 9 wherein the subject is a DS individual.

15. A method according to any preceding claim wherein the step of detecting the presence or absence of intronic polymorphisms or of DNA encoding such isoforms is carried out *ex vivo.*

16. A method according to claim 15 wherein said detection step involves collecting a sample of biological material containing DNA from the subject.

17. A method according to claim 16, wherein the biological sample is blood.

18. A method according to claim 15 wherein said detection step involves collecting a sample of biological material containing PS-1 from the subject.

19. A method according to claim 18, wherein the biological sample is cerebrospinal fluid.

20. A method of treatment of β amyloid-related disease by the supplementation of PS-1 protein expression.

21. A method according to claim 20 wherein said supplementation is effected by direct administration of PS-1 isoform or by gene therapy.

22. A method according to claim 20, wherein said supplementation is effected by the generation of an agonist which mimics the action of PS-1 on the PS-1 receptors.
